# EUROPEAN PATENT SPECIFICATION

(11) **EP 2 828 232 B1**
(45) Date of publication and mention of the grant of the patent: **20.04.2016**
(21) Application number: 13710583.9
(22) Date of filing: 25.02.2013
(51) Int. Cl.: C07C 67/38, C07C 69/54

(54) **PROCESS FOR PRODUCING ALKYL METHACRYLATE**
VERFAHREN ZUR HERSTELLUNG VON ALKYLMETHACRYLAT
PROCÉDÉ DE PRODUCTION DE MÉTHACRYLATE D'ALKYLE

(30) Priority: 21.03.2012 JP 2012063289
(43) Date of publication of application: 28.01.2015
(73) Proprietor: Sumitomo Chemical Company Limited, Tokyo 104-8260 (JP)
(72) Inventor: KANAZAWA, Hideo, Niihama-shi Ehime 792-8521 (JP); KAWAMURA, Masato, Niihama-shi Ehime 792-8521 (JP); MIURA, Naoki, Niihama-shi Ehime 792-8521 (JP)
(74) Representative: Henkel, Breuer & Partner
(86) International application number: PCT/JP2013/055759
(87) International publication number: WO 2013/141004

(56) References cited:
- US-A- 4 739 109
- US-A- 5 869 738
- US-A1- 2011 046 417

## Description

### TECHNICAL FIELD

The present invention relates to a process for producing an alkyl methacrylate.

### BACKGROUND ART

JP-A-2010-120921 (Patent Document 1) discloses a process for producing an alkyl methacrylate, specifically a process for producing methyl methacrylate by reacting methylacetylene with carbon monoxide and methanol in the presence of a catalyst comprising a palladium compound, a protonic acid and a phosphine compound.

### CITATION LIST

### PATENT DOCUMENT

Patent Document 1: JP-A-2010-120921

### SUMMARY OF THE INVENTION

However, the aforementioned previous process is not necessarily satisfactory in respect of productivity based on the amount of the palladium metal used.

Then, an object of the present invention is to provide a process for efficiently producing an alkyl methacrylate.

The present inventors intensively studied and, as a result, found that a small amount of water is usually contained in a lower alcohol, such as methanol, to be used for the production of an alkyl methacrylate, and this water influences on the activity of catalyst to be used for the production. The present inventors also found that the aforementioned problems can be solved by adjusting this water amount to a certain prescribed amount or less relative to the catalyst, which resulted in the completion of the present invention.

That is, the present invention is directed to a process for producing an alkyl methacrylate comprising reacting carbon monoxide and an alcohol having 1 to 3 carbon atoms with methylacetylene in the presence of a catalyst comprising a Group 10 metal compound, a protonic acid and a phosphine compound, wherein
the methylacetylene has a propadiene content of 50 ppm by weight or less, the use amount of the methylacetylene is 200000 mol or more based on 1 mol of the Group 10 metal compound, and the reaction is performed in the presence of water in an amount of 40000 mol or less based on 1 mol of the Group 10 metal compound.

In one embodiment, the reaction is performed in the presence of water in an amount of 4000 mol or less based on 1 mol of the Group 10 metal compound.

In another embodiment, the reaction is performed in the presence of water in an amount of 1000 mol or less based on 1 mol of the group 10 metal compound.

In another embodiment, the alcohol having 1 to 3 carbon atoms contains water in an amount of 1000 ppm by weight or less.

In another embodiment, the amount of the water contained in the alcohol having 1 to 3 carbon atoms is 500 ppm by weight or less.

In another embodiment, the use amount of the phosphine compound is 0.000020 mol or more based on 1 mol of the methylacetylene.

In another embodiment, the catalyst further comprises an amine compound.

In another embodiment, the use amount of the phosphine compound is 2 mol or more based on 1 mol of the propadiene.

In another embodiment, the Group 10 metal compound is a palladium compound.

In another embodiment, the phosphine compound is an aromatic tertiary pyridylphosphine.

In another embodiment, the protonic acid is a sulfonic acid.

In another embodiment, the amine compound is N,N-dimethylaniline.

According to the present invention, an alkyl methacrylate can be produced efficiently.

### DESCRIPTION OF PREFERRED EMBODIMENTS

In the present invention, carbon monoxide and an alcohol having 1 to 3 carbon atoms are reacted with methylacetylene in the presence of a catalyst comprising a Group 10 metal compound, a protonic acid and a phosphine compound.

Although the methylacetylene may contain propadiene in the process of the present invention, the content of the propadiene in the methylacetylene to be used is 50 ppm by weight or less, preferably 30 ppm by weight or less, more preferably 20 ppm by weight or less, further preferably 10 ppm by weight or less, and most preferably 5 ppm by weight or less. Methylacetylene may contain impurities other than propadiene, as far as they do not remarkably inhibit the reaction. Examples of such impurities include butadiene, propylene, butene, propane, carbon monoxide, and carbon dioxide. Carbon monoxide to be used in the present invention may be one containing gases such as nitrogen, helium, carbon dioxide, and argon which are inert to a catalyst and methylacetylene, as well as pure carbon monoxide.

The reaction of the present invention is carried out in the presence of a catalyst comprising a Group 10 metal compound, a protonic acid and a phosphine compound. Usually, the catalyst is used in the form of a mixture of the Group 10 metal compound, the protonic acid and the phosphine compound. The use amount of the Group 10 metal compound, the protonic acid and the phosphine compound may be a catalytic amount, but typically they are used in a mode described as an example below.

Examples of the Group 10 metal compound include a nickel compound, a palladium compound, and a platinum compound, and the Group 10 metal compound is preferably a palladium compound. Examples of such a palladium compound include palladium acetylacetonate, tetrakis(triphenylphosphine)palladium, bis(triphenylphosphine)palladium acetate, palladium acetate, palladium trifluoroacetate, palladium trifluoromethanesulfonate, palladium sulfate, palladium chloride and mixtures thereof. The palladium compound is more preferably palladium acetylacetonate, tetrakis(triphenylphosphine)palladium, bis(triphenylphosphine)palladium acetate, palladium acetate, palladium trifluoroacetate, palladium trifluoromethanesulfonate, palladium sulfate and mixtures thereof, and further preferably palladium acetate. The use amount of the Group 10 metal compound is 1/200000 mol or less, and preferably in the range of 1/1000000 to 1/200000 mol based on 1 mol of the methylacetylene to be used. That is, the use amount of methylacetylene is 200000 mol or more, and preferably in the range of 200000 to 1000000 mol based on 1 mol of the Group 10 metal compound to be used.

The phosphine compound is not particularly limited, but usually, a tertiary phosphine compound is used. Preferably, an aromatic tertiary phosphine compound represented by the following formula (1) is used: 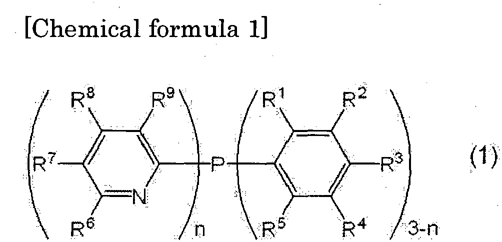 (wherein R¹, R², R³, R⁴, R⁵, R⁶, R⁷, R⁸ and R⁹ are each independently a hydrogen atom or a substituent selected from the group consisting of a halogen atom, a cyano group, a hydroxy group, an alkyl group optionally substituted with a halogen atom, an aralkyl group, a substituted or unsubstituted aryl group, a pyridyl group, a silyl group, an amino group, an alkoxy group, an aralkyloxy group, an aryloxy group and an acyl group, and adjacent groups may be mutually bound to form a ring; and n represents an integer of 0 to 3)

Among the aromatic tertiary phosphine compounds represented by the formula (1), an aromatic tertiary pyridylphosphine compound in which n is an integer of 1 to 3 is more preferable. Examples of the aromatic tertiary pyridylphosphine compound include diphenyl-2-pyridylphosphine, diphenyl(6-methyl-2-pyridyl)phosphine, diphenyl(6-ethyl-2-pyridyl)phosphine, diphenyl[6-(n-propyl)-2-pyridyl]phosphine, diphenyl[6-(iso-propyl)-2-pyridyl]phosphine, diphenyl[6-(n-butyl)-2-pyridyl]phosphine, diphenyl[6-(iso-butyl)-2-pyridyl]phosphine, diphenyl(6-phenyl-2-pyridyl)phosphine, diphenyl(6-hydroxy-2-pyridyl)phosphine, diphenyl(6-methoxy-2-pyridyl)phosphine, diphenyl(6-fluoro-2-pyridyl)phosphine, diphenyl(6-chloro-2-pyridyl)phosphine, diphenyl(6-bromo-2-pyridyl)phosphine, bis(4-fluorophenyl)(2-pyridyl)phosphine, bis(4-chlorophenyl)(2-pyridyl)phosphine, bis(4-bromophenyl)(2-pyridyl)phosphine, bis(3-methylphenyl)(2-pyridyl)phosphine, bis(4-methylphenyl)(2-pyridyl)phosphine, bis(4-methoxyphenyl)(2-pyridyl)phosphine, bis[4-(trifluoromethyl)phenyl](2-pyridyl)phosphine, bis(3,4,5-trifluorophenyl)(2-pyridyl)phosphine, bis(4-fluorophenyl)(6-methyl-2-pyridyl)phosphine, bis(4-chlorophenyl)(6-methyl-2-pyridyl)phosphine, bis(4-bromophenyl)(6-methyl-2-pyridyl)phosphine, bis(3-methylphenyl)(6-methyl-2-pyridyl)phosphine, bis(4-methylphenyl)(6-methyl-2-pyridyl)phosphine, bis(4-methoxyphenyl)(6-methyl-2-pyridyl)phosphine, bis[4-(trifluoromethyl)phenyl](6-methyl-2-pyridyl)phosphine, bis(4-methylphenyl)(6-ethyl-2-pyridyl)phosphine, bis(4-methylphenyl)[6-(n-propyl)-2-pyridyl]phosphine, bis(4-methylphenyl)[6-(iso-propyl)-2-pyridyl]phosphine, bis(4-methylphenyl)[6-(n-butyl)-2-pyridyl]phosphine, bis(4-methylphenyl)[6-(iso-butyl)-2-pyridyl]phosphine, bis(4-methoxyphenyl)(6-ethyl-2-pyridyl)phosphine, bis(4-methoxyphenyl)[6-(n-propyl)-2-pyridyl]phosphine, bis(4-methoxyphenyl)[6-(iso-propyl)-2-pyridyl]phosphine, bis(4-methoxyphenyl)[6-(n-butyl)-2-pyridyl]phosphine, bis(4-methoxyphenyl)[6-(iso-butyl)-2-pyridyl]phosphine, bis(2-pyridyl)phenylphosphine, tris(2-pyridyl)phosphine, bis(6-methyl-2-pyridyl)phenylphosphine, tris(6-methyl-2-pyridyl)phosphine, bis(3,5-dimethylphenyl)(2-pyridylphosphine), bis(3,5-dimethylphenyl)(6-methyl-2-pyridylphosphine), bis(3,5-dimethylphenyl)(6-ethyl-2-pyridylphosphine), bis(3,5-dimethyl-4-methoxyphenyl)(2-pyridylphosphine), bis(3,5-dimethyl-4-methoxyphenyl)(6-methyl-2-pyridylphosphine), and bis(3,5-dimethyl-4-methoxyphenyl)(6-ethyl-2-pyridylphosphine), and further preferably, diphenyl-2-pyridylphosphine, diphenyl(6-methyl-2-pyridyl)phosphine, bis(3,5-dimethylphenyl)(2-pyridylphosphine), bis(3,5-dimethylphenyl)(6-methyl-2-pyridylphosphine), bis(3,5-dimethyl-4-methoxyphenyl)(2-pyridylphosphine), and bis(3,5-dimethyl-4-methoxyphenyl)(6-methyl-2-pyridylphosphine).

These aromatic tertiary pyridyl phosphine compounds are not limited to single use, and they may be appropriately used in the form of a mixture.

The use amount of the phosphine compound is preferably 2 to 300 mol, and more preferably 5 to 240 mol based on 1 mol of the Group 10 metal compound to be used. In addition, the use amount of the phosphine compound is preferably 0.000020 mol or more, and more preferably 0.000020 to 0.00048 mol based on 1 mol of the methylacetylene to be used, and the use amount of the phosphine compound based on 1 mol of the propadiene to be used is preferably 2 mol or more.

The aromatic tertiary pyridylphosphine compound can be produced by a known method. For example, as disclosed in JP-A-2-277551, a variety of aromatic tertiary pyridylphosphine compounds can be produced by lithiation by reacting a halogenated pyridine with an alkyllithium to lithiate it and, thereafter, reacting the resultant with phosphine chloride.

Also when a monodentate tertiary monophosphine compound is made to coexist with the aromatic tertiary pyridylphosphine compound, a preferable result is obtained. The monodentate tertiary monophosphine compound in this case is a tertiary phosphine compound having no other functional group which serves as a coordination group than one phosphorus atom. Examples of the monodentate tertiary monophosphine compound include a trialkylphosphine compound, or an aromatic tertiary phosphine compound having the same or different aryl groups, which may be substituted with one or more substituent selected from the group consisting of an alkyl group, a halogen atom, a haloalkyl group and an alkoxy group. Examples of such monodentate tertiary monophosphine compound include triethylphosphine, tributylphosphine, tricyclohexylphosphine, triphenylphosphine, tris(4-fluorophenyl)phosphine, tris(4-chlorophenyl)phosphine, tris(4-methylphenyl)phosphine, tris[4-(trifluoromethyl)phenyl]phosphine, tris(4-methoxyphenyl)phosphine, tris(3-methylphenyl)phosphine, tris[3,5-bis(trifluoromethyl)phenyl]phosphine, (4-methylphenyl)(diphenyl)phosphine and mixtures thereof, and the aromatic tertiary phosphine compound is preferably used, and triphenylphosphine is further preferably used. The use amount thereof is not particularly limited, but it is preferably in the range of 1 to 600 mol, and more preferably in the range of 1 to 300 mol based on 1 mol of the Group 10 metal compound to be used.

Examples of the protonic acid include organic or inorganic protonic acids. Examples of the protonic acid include boric acid, orthophosphoric acid, pyrophosphoric acid, sulfuric acid, hydrohalic acid, benzenephosphoric acid, benzenesulfonic acid, p-toluenesulfonic acid, naphthalenesulfonic acid, chlorosulfonic acid, methanesulfonic acid, trifluoromethanesulfonic acid, trimethylmethanesulfonic acid, monochloroacetic acid, dichloroacetic acid, trichloroacetic acid, trifluoroacetic acid, oxalic acid, bis(trifluoromethanesulfonyl)imide, tris(trifluoromethanesulfonyl)methide and mixtures thereof, and sulfonic acids such as benzenesulfonic acid, p-toluenesulfonic acid, naphthalenesulfonic acid, chlorosulfonic acid, methanesulfonic acid, trifluoromethanesulfonic acid, and trimethylmethanesulfonic acid are preferably used, and methanesulfonic acid is further preferably used. While the use amount thereof is not particularly limited, usually, 3 to 300 mol based on 1 mol of the Group 10 metal compound is sufficient. More preferably, the amount is 10 to 240 mol based on 1 mol of the Group 10 metal compound.

In the reaction, the use of an amine compound as a catalyst component is not essential, but a reaction in the presence of the amine compound may give a preferable result. The amine compound to be used is not particularly limited, but usually, a known tertiary amine or cyclic amine is used. Examples of the amine compound include N,N-dialkylaniline, pyridine, quinoline, isoquinoline, triazine, imidazole, triethylamine, tributylamine, N,N-diisopropylethylamine and mixtures thereof, and N,N-dimethylaniline and pyridine are preferable. The addition amount of the amine compound is not particularly limited, but it is preferably in the range of 1 to 50 mol, and more preferably in the range of 1 to 10 mol based on 1 mol of the protonic acid to be used.

Examples of the alcohol having 1 to 3 carbon atoms to be used in the present invention include methanol, ethanol, 1-propanol, 2-propanol, and ethylene glycol. In one preferable embodiment, methyl methacrylate is produced by a reaction with methanol. The use amount of the alcohol is appropriately adjusted according to the amount of water contained in the alcohol, so that the amount of water present in the reaction system becomes 40000 mol or less based on 1 mol of the Group 10 metal compound. The amount of water contained in the alcohol is preferably 1000 ppm by weight or less, more preferably 750 ppm by weight or less, further preferably 500 ppm by weight or less, and particularly preferably 100 ppm by weight or less. The use amount of the alcohol based on 1 mol of the methylacetylene to be used is preferably 1 mol or more, and more preferably 1 to 5 mol.

In the present invention, the reaction is performed in the presence of water in an amount of 40000 mol or less based on 1 mol of the Group 10 metal compound. The amount of water present in the reaction system is preferably 4000 mol or less, more preferably 1000 mol or less, and further preferably 500 mol or less based on 1 mol of the Group 10 metal compound, and the lower limit thereof is not particularly limited, but it is usually 10 mol or more. The amount of the water present in the reaction system is preferably adjusted by the adjustment of the amount of the water contained in the alcohol having 1 to 3 carbon atoms. The adjustment of the amount of the water contained in the alcohol having 1 to 3 carbon atoms can be performed by, for example, treating the alcohol with a drying agent such as molecular sieve, alumina, silica gel, Na₂SO₄, MgSO₄, CuSO₄, P₂O₅, CaH₂, BaO, or CaO to decrease the amount of the water contained in the alcohol having 1 to 3 carbon atoms.

While the use of a solvent is not essential in the reaction of the present invention, since it is preferable to reduce the partial pressure of methylacetylene and propadiene, an alcohol having 1 to 3 carbon atoms is preferably used excessively in place of the solvent. However, a solvent other than the alcohol may also be used. A usable solvent and the use amount thereof are not particularly limited as far as water is present in the reaction in an amount of 40000 mol or less based on 1 mol of the Group 10 metal compound, and an alkoxycarbonylation reaction is not greatly inhibited. Examples of the solvent include aromatic hydrocarbons, aliphatic hydrocarbons, sulfoxides, sulfones, esters, ketones, ethers, amides, alcohols, ionic liquids and mixtures thereof. Specific examples of the solvent include toluene, xylene, hexane, cyclohexane, heptane, octane, dimethyl sulfoxide, sulfolane, methyl acetate, ethyl acetate, methyl acrylate, ethyl acrylate, methyl methacrylate, ethyl methacrylate, acetone, methyl ethyl ketone, anisole, dimethoxyethane, diethyl ether, tetrahydrofuran, diglyme, dibutyl ether, dimethylformamide, N-methylpyrrolidone, dimethylacetamide, butanol and mixtures thereof.

In the reaction, the reaction temperature is not particularly limited, but preferably, the reaction is performed at a temperature in the range of 20 to 100°C. In addition, the reaction time, which may depend on reaction conditions such as the use amount of the catalyst, the reaction temperature, and the reaction pressure, is usually 0.5 to 48 hours. The reaction pressure in the reaction is not particularly limited, but it is preferably in the range of 0.5 to 10 MPaG (gauge pressure), and more preferably in the range of 1.0 to 7 MPaG (gauge pressure). The partial pressure of carbon monoxide thereupon is not particularly limited, but it is preferably in the range of 0.5 to 10 MPaG (gauge pressure), and more preferably in the range of 1.0 to 7 MPaG (gauge pressure). The mode of the reaction of the present invention is not particularly limited, and the reaction may be carried out either by a batch system or a continuous system.

Examples of an alkyl methacrylate to be obtained in the reaction include methyl methacrylate, ethyl methacrylate, n-propyl methacrylate, isopropyl methacrylate, and 2-hydroxyethyl methacrylate.

### EXAMPLES

The present invention will be explained by way of examples, but the present invention is not limited to these examples.

### PRODUCTION EXAMPLE 1

In a nitrogen-substituted 1000 ml Schlenk flask, 184 mg (0.80 mmol) of palladium acetate and 3.276 g (12.1 mmol) of diphenyl-2-pyridylphosphine were dissolved in 350 ml of methanol, 3.574 g (18.6 mmol) of paratoluenesulfonic acid monohydrate was added, and the mixture was stirred at room temperature to obtain a catalyst solution. The resulting catalyst solution was introduced into a nitrogen-substituted 1100 ml autoclave, and was cooled in an ethanol/dry ice bath. Into the cooled autoclave was introduced 219 g (5.4 mol, propadiene content: 15 to 23 ppm by weight) of methylacetylene, the autoclave was pressurized with carbon monoxide, and the pressure was retained at 1.0 MPaG (gauge pressure) (during the reaction, carbon monoxide in an amount corresponding to the consumed amount was constantly introduced with a pressure reducing valve so that the total pressure became 1.0 MPaG (gauge pressure)). After stirring at 50°C for 2.5 hours, the reaction liquid was cooled again in an ethanol/dry ice bath. After unreacted carbon monoxide was released, the reaction liquid was heated to 35°C, and vaporized methylacetylene gas was collected in the form of liquid in a Schlenk flask which had been cooled in an ethanol/dry ice bath in advance. The liquid was filled into a cylinder as it was to obtain 83 g of methylacetylene having a propadiene content of 1 ppm by weight.

### EXAMPLE 1

After 2.7 mg (0.0118 mmol) of palladium acetate and 200 mg (0.707 mmol) of diphenyl(6-methyl-2-pyridyl)phosphine were dissolved in 50 mL of methanol (water content: 53 ppm by weight) in a Schlenk flask, 0.23 ml (1.77 mmol) of N,N-dimethylaniline and 70 µl (1.06 mmol) of methanesulfonic acid were added to prepare a catalyst solution. This catalyst solution (1.26 ml) (corresponding to 0.000295 mmol of palladium, 0.0177 mmol of diphenyl(6-methyl-2-pyridyl)phosphine) was taken, and introduced into an autoclave made of stainless steel having an internal volume of 230 ml under a nitrogen atmosphere and, thereafter, 29 ml of methanol (water content: 53 ppm by weight) was further introduced.

The autoclave containing the catalyst solution was cooled under an ethanol/dry ice bath, 10.6 g of methylacetylene (methylacetylene content: 259 mmol, propadiene content: 0.0008 mmol) having a propadiene concentration of 3 ppm was introduced, thereafter, the system was pressurized with carbon monoxide (CO), and the pressure was retained at 1.5 MPaG (gauge pressure). The molar ratio of water/palladium present in the reaction system was 230, and the molar ratio of methanol/methylacetylene used in the reaction was 2.9. During the reaction, carbon monoxide in an amount corresponding to the consumed amount was constantly introduced with a pressure reducing valve in order to maintain the CO partial pressure at 1.5 MPaG (gauge pressure),. When the reaction liquid after being retained at a reaction temperature of 50°C for 6.7 hours was quantitatively analyzed by gas chromatography (GC), the production amount of methyl methacrylate was 7.3 × 10⁵ mol/Pd mol.

### EXAMPLE 2

When the same operation as that of Example 1 was performed except that methanol (water content: 500 ppm by weight) was used in place of the methanol (water content: 53 ppm by weight), and 10.2 g of methylacetylene (methylacetylene content: 253 mmol, propadiene content: 0.0003 mmol) having a propadiene concentration of 1 ppm was used in place of 10.6 g of the methylacetylene having a propadiene concentration of 3 ppm, the production amount of methyl methacrylate was 7.1 × 10⁵ mol/Pd mol. The molar ratio of water/palladium present in the reaction system was 2200, and the molar ratio of methanol/methyl acetylene used in the reaction was 3.0.

### COMPARATIVE EXAMPLE 1

When the same operation as that of Example 1 was performed except that methanol (water content: 1.0 wt%) was used in place of the methanol (water content: 53 ppm by weight), and 10.8 g of methylacetylene (methylacetylene content: 265 mmol, propadiene content: 0.0005 mmol) having a propadiene concentration of 2 ppm was used in place of 10.6 g of the methylacetylene having a propadiene concentration of 3 ppm, the production amount of methyl methacrylate was 5.6 × 10⁵ mol/Pd mol. The molar ratio of water/palladium present in the reaction system was 45000, and the molar ratio of methanol/methylacetylene used in the reaction was 2.8.

The results obtained in the examples and the comparative example are shown in Table 1.

**[Table 1]**

| | Example 1 | Example 2 | Comparative Example 1 |
|---|---|---|---|
| Water content in methanol (ppm by weight) | 53 | 500 | 10000 |
| Use amount of palladium (mmol) | 0.000295 | 0.000295 | 0.000295 |
| Use amount of phosphine compound (mmol) | 0.0177 | 0.0177 | 0.0177 |
| Use amount of methylacetylene (mmol) | 259 | 253 | 265 |
| Methylacetylene/palladium (molar ratio) | 8.8 × 10⁵ | 8.6 × 10⁵ | 9.0 × 10⁵ |
| Water/palladium (molar ratio) | 230 | 2200 | 45000 |
| Phosphine compound/methylacetylene (molar ratio) | 0.000068 | 0.000070 | 0.000067 |
| Production amount of methyl methacrylate (mol/Pd mol) | 7.3 × 10⁵ | 7.1 × 10⁵ | 5.6 × 10⁵ |

### INDUSTRIAL APPLICABILITY

The present invention can be utilized for the production of an alkoxycarbonyl compound, specifically, an alkyl methacrylate, particularly methyl methacrylate.

## Claims

1. A process for producing an alkyl methacrylate comprising reacting carbon monoxide and an alcohol having 1 to 3 carbon atoms with methylacetylene in the presence of a catalyst comprising a Group 10 metal compound, a protonic acid and a phosphine compound, wherein
the methylacetylene has a propadiene content of 50 ppm by weight or less, the use amount of the methylacetylene is 200000 mol or more based on 1 mol of the Group 10 metal compound, and the reaction is performed in the presence of water in an amount of 40000 mol or less based on 1 mol of the Group 10 metal compound.

2. The process according to claim 1, wherein the reaction is performed in the presence of water in an amount of 4000 mol or less based on 1 mol of the Group 10 metal compound.

3. The process according to claim 1, wherein the reaction is performed in the presence of water in an amount of 1000 mol or less based on 1 mol of the Group 10 metal compound.

4. The process according to any one of claims 1 to 3, wherein the alcohol having 1 to 3 carbon atoms contains water in an amount of 1000 ppm by weight or less.

5. The process according to any one of claims 1 to 3, wherein the water contained in the alcohol having 1 to 3 carbon atoms is 500 ppm by weight or less.

6. The process according to any one of claims 1 to 5, wherein the use amount of the phosphine compound is 0.000020 mol or more based on 1 mol of the methylacetylene.

7. The process according to any one of claims 1 to 6, wherein the catalyst further comprises an amine compound.

8. The process according to any one of claims 1 to 7, wherein the use amount of the phosphine compound is 2 mol or more based on 1 mol of the propadiene.

9. The process according to any one of claims 1 to 8, wherein the Group 10 metal compound is a palladium compound.

10. The process according to any one of claims 1 to 9, wherein the phosphine compound is an aromatic tertiary pyridylphosphine.

11. The process according to any one of claims 1 to 10, wherein the protonic acid is a sulfonic acid.

12. The process according to any one of claims 7 to 11, wherein the amine compound is N,N-dimethylaniline.

## Patentansprüche

1. Verfahren zur Herstellung eines Alkylmethacrylats, umfassend das Umsetzen von Kohlenmonoxid und eines 1 bis 3 Kohlenstoffatome aufweisenden Alkohols mit Methylacetylen in Gegenwart eines Katalysators, welcher eine Verbindung eines Gruppe 10-Metalls umfasst, einer Protonensäure sowie einer Phosphinverbindung, wobei
das Methylacetylen 50 Gew.-ppm oder weniger an Propadien enthält, die eingesetzte Menge an Methylacetylen 200000 Mol oder mehr, bezogen auf 1 Mol der Verbindung des Gruppe 10-Metalls, beträgt, und die Reaktion in Gegenwart einer Menge von 40000 Mol oder weniger an Wasser, bezogen auf 1 Mol der Verbindung des Gruppe 10-Metalls, durchgeführt wird.

2. Verfahren nach Anspruch 1, wobei die Reaktion in Gegenwart einer Menge von 4000 Mol oder weniger an Wasser, bezogen auf 1 Mol der Verbindung des Gruppe 10-Metalls, durchgeführt wird.

3. Verfahren nach Anspruch 1, wobei die Reaktion in Gegenwart einer Menge von 1000 Mol oder weniger an Wasser, bezogen auf 1 Mol der Verbindung des Gruppe 10-Metalls, durchgeführt wird.

4. Verfahren nach einem der Ansprüche 1 bis 3, wobei der 1 bis 3 Kohlenstoffatome aufweisende Alkohol eine Menge von 1000 Gew.-ppm oder weniger an Wasser enthält.

5. Verfahren nach einem der Ansprüche 1 bis 3, wobei 500 Gew.-ppm oder weniger an Wasser in dem 1 bis 3 Kohlenstoffatome aufweisenden Alkohol enthalten sind.

6. Verfahren nach einem der Ansprüche 1 bis 5, wobei die eingesetzte Menge an Phosphinverbindung 0.000020 Mol oder mehr, bezogen auf 1 Mol des Methylacetylens, beträgt.

7. Verfahren nach einem der Ansprüche 1 bis 6, wobei der Katalysator weiterhin eine Aminverbindung umfasst.

8. Verfahren nach einem der Ansprüche 1 bis 7, wobei die eingesetzte Menge an Phosphinverbindung 2 Mol oder mehr, bezogen auf 1 Mol des Propadiens, beträgt.

9. Verfahren nach einem der Ansprüche 1 bis 8, wobei die Verbindung des Gruppe 10-Metalls eine Palladiumverbindung ist.

10. Verfahren nach einem der Ansprüche 1 bis 9, wobei die Phosphinverbindung ein aromatisches tertiäres Pyridylphosphin ist.

11. Verfahren nach einem der Ansprüche 1 bis 10, wobei die Protonensäure eine Sulfonsäure ist.

12. Verfahren nach einem der Ansprüche 7 bis 11, wobei die Aminverbindung N,N-Dimethylanilin ist.

## Revendications

1. Procédé de préparation d'un méthacrylate d'alkyle, dans lequel on fait réagir du monoxyde de carbone et un alcool ayant de 1 à 3 atomes de carbone sur du méthylacétylène en la présence d'un catalyseur comprenant un composé d'un métal du groupe 10, un acide protonique et une phosphine, dans lequel
le méthylacétylène a une teneur en propadiène inférieure ou égale à 50 parties par million en poids, la quantité du méthylacétylène utilisée est supérieure ou égale à 200 000 moles pour une mole du composé du métal du groupe 10 et on effectue la réaction en la présence d'eau en une quantité inférieure ou égale à 40 000 moles pour une mole du composé du métal du groupe 10.

2. Procédé suivant la revendication 1, dans lequel on effectue la réaction en la présence d'eau en une quantité inférieure ou égale à 4 000 moles pour une mole du composé du métal du groupe 10.

3. Procédé suivant la revendication 1, dans lequel on effectue la réaction en la présence d'eau en une quantité inférieure ou égale à 1 000 moles pour une mole du composé du métal du groupe 10.

4. Procédé suivant l'une quelconque des revendications 1 à 3, dans lequel l'alcool ayant de 1 à 3 atomes de carbone contient de l'eau en une quantité inférieure ou égale à 1 000 parties par million en poids.

5. Procédé suivant l'une quelconque des revendications 1 à 3, dans lequel l'eau contenue dans l'alcool ayant de 1 à 3 atomes de carbone est inférieure ou égale à 500 parties par million en poids.

6. Procédé suivant l'une quelconque des revendications 1 à 5, dans lequel la quantité de la phosphine utilisée est supérieure ou égale à 0,000020 mole par mole du méthylacétylène.

7. Procédé suivant l'une quelconque des revendications 1 à 6, dans lequel le catalyseur comprend en outre une amine.

8. Procédé suivant l'une quelconque des revendications 1 à 7, dans lequel la quantité de la phosphine qui est utilisée est supérieure ou égale à 2 moles par mole du propadiène.

9. Procédé suivant l'une quelconque des revendications 1 à 8, dans lequel le composé du métal du groupe 10 est un composé du palladium.

10. Procédé suivant l'une quelconque des revendications 1 à 9, dans lequel la phosphine est une pyridylphosphine tertiaire aromatique.

11. Procédé suivant l'une quelconque des revendications 1 à 10, dans lequel l'acide protonique est l'acide sulfonique.

12. Procédé suivant l'une quelconque des revendications 7 à 11, dans lequel l'amine est la N,N-diméthylaniline.
